# FASCICULE DE BREVET EUROPEEN

(11) **EP 3 810 790 B1**
(45) Date de publication et mention de la délivrance du brevet: **02.04.2025**
(21) Numéro de dépôt: 19746529.7
(22) Date de dépôt: 24.06.2019
(51) Int. Cl.: C12Q 1/04, C12Q 1/22, G01N 1/38, G01N 1/02

(54) **PROCÉDÉ DE DÉTECTION ET/OU D'IDENTIFICATION D'AU MOINS UN MICROORGANISME CIBLE PRÉSENT SUR UNE SURFACE**
VERFAHREN ZUM NACHWEIS UND/ODER ZUR IDENTIFIZIERUNG MINDESTENS EINES AUF EINER OBERFLÄCHE VORHANDENEN ZIELMIKROORGANISMUS
METHOD FOR DETECTING AND/OR IDENTIFYING AT LEAST ONE TARGET MICROORGANISM PRESENT ON A SURFACE

(30) Priorité: 25.06.2018 FR 1855641
(43) Date de publication de la demande: 28.04.2021
(73) Titulaire: bioMérieux, 69280 Marcy l'Etoile (FR)
(72) Inventeur: GROSSELIN, Jeanne, 26750 Saint Paul Les Romans (FR); LEBLANC, Laurent, 69730 Genay (FR); MENESSIER, Sébastien, 42800 Rive De Gier (FR); RAYMOND, Jean-Claude, 69690 Bessenay (FR); MAO, Bosi, 69007 Lyon (FR)
(74) Mandataire: bioMérieux PI Groupement mandataires
(86) Numéro de dépôt international: PCT/FR2019/051534
(87) Numéro de publication internationale: WO 2020/002806

(56) Documents cités:
- WO-A1-2018/043636
- WO-A2-2015/083181
- US-A- 4 237 223
- DOMINIQUE JASMIN LUNTER ET AL: "New film forming emulsions containing Eudragit NE and/or RS 30D for sustained dermal delivery of nonivamide", EUROPEAN JOURNAL OF PHARMACEUTICS AND BIOPHARMACEUTICS., vol. 82, no. 2, 28 June 2012 (2012-06-28), NL, pages 291 - 298, XP055556581, ISSN: 0939-6411, DOI: 10.1016/j.ejpb.2012.06.010

## Description

### Domaine technique

La présente invention concerne le domaine du contrôle microbiologique. Plus particulièrement, la présente invention a trait à une méthode de détection et/ou d'identification d'au moins un microorganisme cible présent sur une surface.

### Etat de la technique

Le contrôle microbiologique est un facteur critique, notamment dans les milieux industriels et hospitaliers. Dans les industries pharmaceutiques, cosmétiques ou agro-alimentaires, le diagnostic permet ainsi la production de produits sûrs en conformité avec les normes et directives internationales. La fabrication de ces produits nécessite des contrôles très sévères pour garantir leur qualité microbiologique et leur composition. Ces tests de contrôle microbiologique sont réalisés tout au long de la chaîne de production, de la matière première au produit fini et permettent de vérifier par exemple l'absence de bactéries pathogènes, la stérilité (aucun microorganisme ne doit être présent), ou encore la non-prolifération d'une bactérie commensale (normalement présente chez l'homme et banale en faible concentration) au-delà d'un certain seuil. L'environnement de production (air, eau, surfaces) est également contrôlé régulièrement par des tests de diagnostic. Les réglementations imposent que certains produits, comme les médicaments injectables, soient stériles lors de leur commercialisation. Cette garantie de stérilité est apportée par des tests sur les matières premières, les produits en cours de fabrication, l'environnement de production et les produits finis. La rigueur de ces contrôles est un gage de qualité : elle garantit la sécurité du consommateur.

Différents systèmes ou dispositifs ont été développés pour le contrôle de l'air, des liquides et des surfaces. Plusieurs sociétés ont développé des milieux de culture qui assurent une croissance des microorganismes prélevés directement sur les surfaces contaminées, et permettent une lecture directe du nombre de colonies de la boîte. On peut citer à titre d'exemple les films enduits de gélose de type Pétrifilm^{®} ou encore les boîtes Count-Tact^{®}. Les prélèvements sont effectués par application, après enlèvement du couvercle de la boîte, par pression manuelle, sur le fond de la boîte, pendant un temps non défini, de façon à ce que le milieu gélose entre en contact le plus étroit possible avec la surface à prélever. Un inconvénient majeur de cette méthode dite d'empreinte par les milieux de cultures réside dans le fait d'introduire dans des zones sensibles tels que des zones à atmosphère contrôlée de la matière organique pouvant être source de contamination. Un défaut de reproductibilité des résultats peut également être observé, principalement dû à des variations de contact entre le milieu gélose et la surface à contrôler, en raison des différences de la force manuelle appliquée sur la boîte qui peut considérablement varier d'un opérateur à l'autre, voire avec un même opérateur lors de différents prélèvements.

Une autre méthode pour contrôler les surfaces est celle dite par frottis, qui est basée sur le décrochement des micro-organismes par frottement de la surface. Cette action peut être réalisée par une chiffonnette ou un écouvillon sec ou humide. L'écouvillonnage humide est une méthode de prélèvement plus efficace que l'écouvillonnage à sec, la supériorité de l'écouvillonnage humide s'expliquant par un contact support/écouvillon amélioré par la présence d'eau. Néanmoins l'écouvillonnage présente l'inconvénient d'un faible rendement puisqu'il nécessite à la fois l'arrachement des bactéries du support par l'écouvillon puis du relargage des bactéries de l'écouvillon vers le liquide. D'un point de vue reproductibilité, il est assez difficile de standardiser un écouvillonnage dont la réalisation sera dépendante du manipulateur.

Le document WO2018/043636 décrit une méthode de détection de bactéries cutanées comprenant l'application sur la peau d'une solution comprenant un polymère de type alcool polyvinylique pour former un film mince et la collecte du film obtenu. Dans un des exemples, la présence de bactéries est détectée après dissolution du film dans une solution aqueuse de tampon physiologique salin puis dépôt de la solution sur un milieu de culture gélosé.

La robustesse des différentes méthodes de prélèvement a un impact certain sur la sensibilité, la reproductibilité des contrôles microbiologiques. Il y a donc un réel besoin de fournir une nouvelle méthode de prélèvement d'échantillon.

### Exposé de l'invention

La présente invention se propose de résoudre les problèmes techniques abordés ci-dessus.

Ainsi, un objectif de la présente invention est donc de fournir un procédé de prélèvement, sur une surface, de microorganismes en vue de leur détection sans amener un milieu nutritif sur le lieu de prélèvement. Cet objectif s'avère d'autant plus intéressant dans les zones à atmosphère contrôlé.

Un autre objectif de la présente invention est de fournir un procédé de prélèvement de microorganismes ayant un bon taux de récupération.

Un autre objectif de la présente invention est de fournir un procédé de prélèvement reproductible nécessaire au suivi de contrôle qualité.

Ces objectifs parmi d'autres, sont atteints par la présente invention qui concerne un procédé de détection et/ou d'identification d'au moins un microorganisme cible présent sur une surface, comprenant les étapes suivantes :
a) déposer sur ladite surface, une composition comprenant un polymère synthétique hydrosoluble filmogène, ladite composition étant une composition liquide et/ou visqueuse ou une composition mousseuse ;
b) sécher la composition pour permettre la formation d'un film de polymère,
c) retirer de la surface ledit film de polymère comprenant ledit au moins un microorganisme cible,
d) dissoudre le film polymère avec un diluant aqueux pour former une solution comprenant ledit au moins un microorganisme, ledit diluant aqueux étant un milieu de culture semi-solide,
e) détecter et/ou identifier ledit au moins un microorganisme cible dans tout ou partie de la solution à l'aide d'au moins un moyen de détection.

Dans le cadre de l'invention, le diluant aqueux est un milieu de culture semi-solide, le film polymère étant dissout par mise en contact avec le milieu de culture semi-solide. En effet, le milieu de culture semi-solide est constitué d'une importante quantité d'eau libre permettant au film polymère de se dissoudre à son contact. La solution formée est donc un milieu de culture semi solide comprenant le film polymère dissout.

Lorsque ladite composition est liquide et/ou visqueuse, l'invention concerne un procédé de détection et/ou d'identification d'au moins un microorganisme cible présent sur une surface, comprenant les étapes suivantes :
a) déposer sur ladite surface, une composition liquide et/ou visqueuse comprenant un polymère synthétique hydrosoluble filmogène,
b) sécher ladite composition pour permettre la formation d'un film de polymère,
c) retirer de la surface ledit film de polymère comprenant ledit au moins un microorganisme cible,
d) dissoudre le film polymère avec un diluant aqueux pour former une solution comprenant ledit au moins un microorganisme, ledit diluant aqueux étant un milieu de culture semi-solide,
e) détecter et/ou identifier ledit au moins un microorganisme cible dans tout ou partie de la solution à l'aide d'au moins un moyen de détection.

Ainsi la composition liquide ou visqueuse présente une texture contrôlée, suffisamment liquide et/ou visqueuse pour pouvoir être appliquée sur la zone à tester et former un film uniforme, mais aussi suffisamment épaisse pour rester sur la zone à tester le temps du séchage et former un film présentant une épaisseur appropriée.

Sa texture liquide et/ou visqueuse lui permet également d'épouser parfaitement la surface sur laquelle elle est déposée, que la surface soit lisse ou rugueuse. Il est reconnu que le contrôle microbiologique de certaines surfaces, notamment celles présentant des aspérités, est particulièrement difficile.

On entend par solution liquide et/ou visqueuse au sens de la présente demande, une solution qui s'écoule sous son propre poids à une température comprise entre 15-25°C.

Dans un mode préféré la composition est une composition mousseuse. La solution de polymère est mélangé avec du CO₂ afin d'obtenir une mousse. L'application d'une mousse génère un effet physique par introduction de bulles gazeuses dans le composé polymère liquide. Ceci permet une meilleur récupération de microorganismes sur des surfaces inertes par rapport à une application de polymère sous forme liquide. Ceci est particulièrement avantageux, sur des surfaces rugueuses telles que des surfaces en inox. La mousse revient à une forme liquide et/ou visqueuse après son dépôt très rapidement en quelques minutes selon la quantité déposée.

Les polymères utilisés dans le cadre de l'invention présentent un certain nombre de caractéristiques permettant d'obtenir les propriétés souhaitées. Selon la présente invention, on entend par "polymère filmogène", un polymère apte à former à lui seul ou en présence d'un agent auxiliaire de filmification, un film continu et adhérant sur un support. Le film formé présente de bonnes propriétés d'adhérence sur les surfaces inertes telles que le verre, le plastique, le polystyrène cristal, l'acier inoxydable, et de bonnes propriétés de résistance à la rupture afin d'être retiré de la surface et transporté.

Le polymère utilisé dans le cadre de l'invention a pour rôle de prélever les microorganismes à des fins de détection. Ce polymère est biocompatible, peu voire non toxique pour les microorganismes. Après avoir été retiré de la surface, le film polymère est dissout dans un diluant aqueux et forme une solution qui sera analysée. Dans la présente invention, le polymère est donc un polymère hydrosoluble. Les polymères hydrosolubles sont bien connus et décrits par la revue « Water soluble polymers for pharmaceutical application, Kadajji and Betageri, Polymers, Dec 2011 ».

Leur solubilité en phase aqueuse dépend notamment de la nature chimique des unités monomériques répétées et du poids du polymère. Elle pourra bien entendu être liée au pH ou à la force ionique de la solution dans laquelle il est dissout.

Dans la présente invention, le polymère est un homopolymère ou copolymère synthétique. De préférence, le polymère hydrosoluble synthétique est choisi parmi le polyéthylène, le polyvinyle, le polyacrylique, le polyoxazoline, leurs dérivés ou un mélange de ces polymères.

Plus préférentiellement, le polymère hydrosoluble est choisi parmi l'oxyde de polyéthylène (PEO), le polyéthylène glycol (PEG), le polyvinylpyrrolidone (PVP), l'alcool polyvinylique (PVA ou PVOH), l'acide polyacrylique (PAA), le polyacrylamide, le poly(2-hydroxypropyl methacrylamide), le poly (2-ethyl-2-oxazoline).

Préférentiellement, le polymère utilisé dans le cadre de l'invention est choisi parmi les polymères suivants :
- l'oxyde de polyéthylène d'un poids moléculaire moyen compris entre 100 000 et 8 000 000 g/mol à des concentrations comprises entre 2 et 30 % (m/v)
- Le polyéthylène glycol d'un poids moléculaire moyen compris entre 600 et 20 000 g/mol à des concentrations comprises entre 2 et 30% (m/v)
- Le polyvinylpyrrolidone d'un poids moléculaire moyen compris entre 10 000 et 1 300 000 g/mol à des concentrations comprises entre 2 et 30% (m/v)
- L'alcool polyvinylique d'un poids moléculaire moyen compris entre 9 000 et 200 000 g/mol à des concentrations comprises entre 2 et 60% (m/v) préférentiellement entre 5 et 50% (m/v) encore plus préférentiellement entre 15 et 35% (m/v)
- L'acide polyacrylique (PAA) d'un poids moléculaire moyen compris entre 450 000 et 4 000 000 g/mol à des concentrations comprises entre 1 et 2% (m/v)
- Le polyacrylamide d'un poids moléculaire moyen compris entre 40 000 et 150 000 g/mol à des concentrations comprises entre 2 et 20% (m/v)
- Le poly(2-hydroxypropyl methacrylamide) d'un poids moléculaire moyen compris entre 30 000 et 50 000 g/mol à des concentrations comprises entre 2 et 30% (m/v)
- Le poly (2-ethyl-2-oxazoline) d'un poids moléculaire moyen de 5 000 g/mol à des concentrations comprises entre 2 et 10% (m/v)

La composition est préparée selon des méthodes connues de l'homme du métier. Le polymère est dissout dans un diluant, généralement de l'eau puis la composition est stérilisée par des méthodes classiques telles que la filtration, l'autoclavage, gaz, irradiation.

De préférence, la composition utilisée dans le cadre de l'invention comprend un tensioactif. Les tensioactifs utilisés dans le cadre de l'invention présentent un certain nombre de caractéristiques permettant d'obtenir les propriétés souhaitées. Ainsi le tensioactif est soluble dans la solution de polymère et permet d'améliorer les propriétés de décollement du film de la surface telle que le plastique, polystyrène cristal, verre, acier inoxydable 316L. Avantageusement, le tensioactif a un faible impact sur la formation du film polymère notamment sur la durée du séchage. Le tensioactif peut également neutraliser l'action inhibitrice des antiseptiques sur les microorganismes présents sur la surface. De préférence, le tensioactif est choisi parmi les tensioactifs anioniques tels que le sodium dodecyl sulfate, l'acide cholique et le sodium de dicyclohexyl sulfosuccinate, les tensioactifs cationiques tels que le Benzalkonium chloride, les tensioactifs non ioniques tels que le diethylene glycol, le polysorbate 80 et la saponine, les tensioactifs zwitterioniques tels que le 3 - (N,N-Dimethyltetradecylammonio) propanesulfonate et le 3- [(3-Cholamidopropyl)dimethylammonio] - 1 -propanesulfonate hydrate.

Préférentiellement le tensioactif est du polysorbate 80. Avantageusement, le polysorbate 80 a une concentration égale ou inférieure à 5%, préférentiellement entre 0,5 à 1%.

La composition peut également comprendre d'autres additifs tels que des agents neutralisants, des agents réducteurs, des agents antioxydants afin d'améliorer la récupération de microorganismes stressés. Des colorants peuvent être également ajoutés afin de mieux visualiser le film de polymère.

Pour améliorer les propriétés filmogènes de la composition, un agent auxiliaire de filmification peut avantageusement être ajouté. Un tel agent auxiliaire de filmification peut être choisi parmi tous les composés connus de l'homme du métier, comme étant susceptible de remplir la fonction recherchée, et être notamment choisi parmi les agents plastifiants et les agents de coalescence du ou des polymère(s) filmogène(s).

Le dépôt et l'étalement de la composition se fera avec ou sans applicateur. Elle peut par exemple être déposée par pipetage puis étalée à l'aide d'une oese stérile. Lorsqu'il s'agit d'une composition mousseuse, la solution de polymère est placé dans un dispositif contenant un gaz (par exemple CO2, air, azote) afin de créer par mélange la composition mousseuse lors du dépôt. La composition mousseuse redevient une composition liquide et/ou visqueuse très rapidement après son dépôt.

La composition utilisée dans le cadre de l'invention est ensuite séchée afin de permettre la formation d'un film polymère. L'épaisseur du film formé est par exemple supérieure à 40 µm, de préférence comprise entre 100 µm et 500 µm, plus préférentiellement comprise entre 100 et 300 µm.

Cette phase de séchage peut être un séchage physique et/ou chimique, en présence de catalyseur ou non. Elle peut être réalisée à température ambiante, à l'air libre ou sous flux d'air. En fonction de la température, de l'humidité de l'air, des dimensions (taille, épaisseur) de la composition déposée sur la surface, ladite composition sera séchée au minimum jusqu'à la formation du film de polymère. Le séchage permet la formation d'un réseau au sein de la composition qui va capturer le microorganisme présent sur la surface. Le temps de séchage sera sensiblement identique que la composition ait été déposée sous forme mousseuse ou sous forme liquide et/ou visqueuse.

Le film polymère utilisé dans le cadre de l'invention est faiblement adhérent, permettant son retrait de la surface sans risque de rupture et laissant ladite surface relativement exempte de résidu du film polymérique. Une fois le film polymère retiré de la surface, il peut être déplacé aisément de la zone de prélèvement à la zone d'analyse sans se rompre ou se déchirer. Préférentiellement, le film de polymère a un module élastique (G') supérieur à 100 000 Pa, mesuré par un rhéomètre HR2 (TA Instrument).

De manière avantageuse, le film polymère comprend un tensioactif qui permet de réduire la force nécessaire au retrait du film du support sur lequel il adhère.

Dans le procédé selon Gelesm la présente invention, le film de polymère dans lequel est capturé le microorganisme est ensuite dissout dans un diluant aqueux pour former une solution qui sera analysée. Ainsi, le procédé selon l'invention a pour avantage de s'affranchir de l'étape de décrochage des microorganismes du support de prélèvement tel que l'étape de désorption des microorganismes d'un écouvillon. Le taux de récupération, à savoir le rapport entre la quantité recueillie de microorganismes et la quantité présente sur une surface, est donc particulièrement optimisé. L'étape de dissolution peut être accélérée par agitation de la solution et du diluant.

Un autre avantage de la présente invention est d'utiliser la solution directement pour la détection des microorganismes. Dans le procédé selon l'invention, le diluant aqueux est un milieu de culture semi-solide qui va dissoudre le film polymère lorsque le film polymère est mis à son contact. Ainsi, dans ce mode de réalisation préféré, la solution est un milieu de culture comprenant le microorganisme à détecter.

Par « milieu de culture », on entend un milieu comprenant tous les éléments nécessaires à la survie et/ou à la croissance des micro-organismes et, en particulier des micro-organismes recherchés (par exemple de l'eau tamponnée peptonée). Le milieu de culture peut contenir d'éventuels additifs, par exemple : des peptones, un ou plusieurs facteurs de croissance, des hydrates de carbone, un ou plusieurs agents sélectifs, des tampons, un ou plusieurs gélifiants, une ou plusieurs vitamines, etc. Ce milieu de culture peut se présenter sous forme liquide ou gélifiée prête à l'emploi, à savoir prête à être ensemencée en tube, en flacon ou sur boîte de Pétri. L'expression « milieu de culture » englobe bien évidemment les milieux et bouillons d'enrichissement. Différents milieux de culture peuvent être utilisés en fonction des exigences des microorganismes que l'on souhaite détecter.

Le procédé selon l'invention peut comprendre une étape d'incubation de la solution, avant ou pendant l'étape de détection, à une température et durant un laps de temps suffisant pour permettre la croissance dudit au moins un micro-organisme.

L'incubation s'effectue, en général, à une température allant de 20 à 52°C durant un laps de temps prédéterminé, par exemple de 6h à 28 jours.

Par « moyen de détection » on entend un moyen de détection permettant de mettre en évidence et/ou de mesurer directement ou indirectement un ou plusieurs paramètres biologiques et/ou physico-chimiques d'un échantillon biologique.

Un moyen de détection classique utilisé en microbiologie consiste en milieux de culture solides, liquides ou semi-solides que l'on ensemence. Ainsi, dans un mode de réalisation préféré, le moyen de détection est le milieu de culture dans lequel a été dissout le film polymère.

Ainsi dans un mode de réalisation préféré, le procédé de détection et/ou d'identification d'au moins un microorganisme cible présent sur une surface comprend les étapes suivantes :
a) déposer sur ladite surface, une composition liquide et/ou visqueuse comprenant un polymère synthétique hydrosoluble filmogène,
b) sécher ladite composition pour permettre la formation d'un film de polymère,
c) retirer de la surface ledit film de polymère comprenant ledit au moins un microorganisme cible,
d) dissoudre le film polymère avec un milieu de culture pour former une solution comprenant ledit au moins un microorganisme, ledit diluant aqueux étant un milieu de culture semi-solide,
e) détecter et/ou identifier ledit au moins un microorganisme cible dans tout ou partie de la solution à l'aide d'au moins un moyen de détection, ledit moyen de détection étant ladite solution.

Dans ce mode de réalisation, le film de polymère hydrosoluble est donc dissout dans un diluant aqueux étant un milieu de culture semi-solide. Dans le cas où, le film est déposé sur milieu de culture semi-solide, l'eau contenue dans la gélose permet de solubiliser le film et celui-ci disparait totalement de la surface du milieu de culture. Ce milieu de culture ensemencé constitue alors la solution utilisée dans le procédé de l'invention. Aucune étape de filtration ou de séparation pour récupérer les microorganismes n'est nécessaire. Les milieux de culture sont ensuite incubés dans les conditions habituelles de culture. L'étape de détection et/ou d'identification est donc effectuée directement à partir de la solution. Après incubation, les colonies isolées peuvent être aisément dénombrées afin de rendre un résultat quantitatif du contrôle de surface. Dans ce mode de réalisation particulier, le milieu de culture joue à la fois le rôle de diluant aqueux et de moyen de détection.

Un aspect décrit concerne donc un procédé de détection et/ou d'identification comprenant les étapes suivantes :
a) déposer sur ladite surface, une composition liquide et/ou visqueuse comprenant un polymère synthétique hydrosoluble filmogène,
b) sécher ladite composition pour permettre la formation d'un film de polymère,
c) retirer de la surface ledit film de polymère comprenant ledit au moins un microorganisme cible,
d) dissoudre le film polymère par mise en contact avec un milieu de culture
e) détecter et/ou identifier ledit au moins un microorganisme cible dans ledit milieu de culture

Préférentiellement, le milieu de culture est semi-solide.

Dans un autre aspect décrit, lorsque la composition est une composition mousseuse, le procédé comprend les étapes suivantes :
a) déposer sur ladite surface une composition mousseuse comprenant un polymère synthétique hydrosoluble filmogène,
b) sécher ladite composition pour permettre la formation d'un film de polymère,
c) retirer de la surface ledit film de polymère comprenant ledit au moins un microorganisme cible,
d) dissoudre le film polymère par mise en contact avec un milieu de culture
e) détecter et/ou identifier ledit au moins un microorganisme cible dans ledit milieu de culture.

Préférentiellement, le milieu de culture est semi-solide.

Dans un autre mode de réalisation, l'étape de détection et/ou d'identification consiste en une méthode de détection des microorganismes cinétique par colorimétrie telle que la technologie BacT/ALERT^{®} . Les micro-organismes présents dans le milieu produisent du CO₂ lors de leur phase de croissance. Le CO₂ produit induit une diminution du pH du milieu de culture. Ce changement de pH induit le virage colorimétrique d'un sensor composé de silicone imprégné de détecteurs d'émulsion liquide (LES), déposé au fond de chaque flacon. Une LED envoie un faisceau lumineux sur le sensor. Une photodiode collecte l'intensité de lumière réfléchie par le sensor sous la forme d'unité de réflectance. Les unités de réflectance sont analysées dans le temps. Ainsi le film de polymère est dissout dans une solution physiologique puis injectée dans un flacon BacT/ALERT^{®} (bioMérieux) pour une lecture cinétique automatique.

Dans un autre mode de réalisation, l'étape de détection et/ou identification consiste en une méthode biochimique colorimétrique miniaturisée sur une carte conçue spécifiquement et nommée Vitek^{®}. Le film de polymère est placé dans un tube à hémolyse puis solubilisé. Les tubes sont chargés avec une carte VITEK^{®}. Le système gère l'incubation et la lecture de chaque carte sans aucune autre intervention. Les résultats sont obtenus dans les 2 à 18 heures après chargement.

Dans un autre mode de réalisation, l'étape de détection et/ou identification consiste en une méthode par cytométrie en flux ou en phase solide. Cette méthode consiste à l'analyse individuelle de cellule par détection optique.

Dans un autre mode de réalisation, l'étape de détection et/ou d'identification consiste en une méthode de dénombrement des microorganismes basée sur la méthode du nombre le plus probable (NPP) telle que la technologie Tempo^{®}. Cette méthode consiste en l'analyse des séries de dilutions de l'échantillon initial. La technologie Tempo miniaturise ce test sur une carte conçue spécifiquement. Le film de polymère est mis en contact avec un milieu de culture deshydraté contenu dans un flacon Tempo^{®} (bioMérieux). Après réhydratation et solubilisation complète, les cartes Tempo^{®} vont être remplies puis incubées avant d'être lues automatiquement par fluorescence.

Dans un autre mode de réalisation, l'étape de détection et/ou d'identification consiste en une méthode de détection des microorganismes par PCR. Dans la technologie Gene-Up^{®}, le film de polymère est mis en contact avec un milieu d'enrichissement. Après enrichissement, l'échantillon est lysé et mis en contact avec des réactifs PCR. La plaque est placée dans l'instrument Gene-Up^{®} pour une détection via la technique de PCR. Une autre technologie de biologie moléculaire applicable peut être la technologie Filmarray^{®}.

Dans un autre mode de réalisation, les immuno-essais constituent une autre des technologies utilisées pour le test de détection. Ils font appel aux caractéristiques immunogènes des micro-organismes recherchés. On citera par exemple le moyen de détection VIDAS^{®}. Le film de polymère est déposé dans un tube de milieu d'enrichissement et incubé. Après enrichissement, l'échantillon est déposé dans une barrette VIDAS^{®} (bioMérieux).

### Brève description des figures

la figure 1 est un graphique représentant en ordonnée la diminution de l'épaisseur de la composition liquide et/ou visqueuse au cours de séchage (en µm) en fonction du temps de séchage (en heure).
la figure 2 est un graphique représentant en ordonnée le module de perte (G'') et le module élastique (G') de la composition liquide et/ou visqueuse exprimés en Pascal en fonction du temps de séchage (en heure).
la figure 3 est un graphique représentant en ordonnée la viscosité de la composition liquide et/ou visqueuse exprimée en mPa par seconde en fonction de la concentration en polymère PVOH exprimée en % (m/v).

### Description détaillée de l'invention

Les exemples ci-après permettront de mieux appréhender la présente invention. Toutefois, ces exemples ne sont donnés qu'à titre illustratif et ne doivent en aucun cas être regardés comme limitant la portée de ladite invention d'une quelconque manière. Les exemples qui ne tombent pas sous la portée du procédé revendiqué ne sont donnés qu'à titre illustratif.

### Exemple 1a : Préparation des composition liquides et/ou visqueuses de polymère

Différentes concentrations en polymère hydrosoluble ont été utilisées : de 2 à 50 % m/v. Différentes concentrations de tensioactifs ont été utilisées : de 0 à 5% v/v ou m/v.
1) Le polymère hydrosoluble est pesé en flacon de verre et dilué en eau déminéralisée
2) L'ensemble est mis sous agitation et chauffé afin de dissoudre complètement le polymère en solution
3) Si présence d'un tensioactif, il est ajouté à la solution par pipetage (si liquide) ou par pesé (si solide)
4) La composition est autoclavée en cycle liquide, avec un plateau à 120°C durant 16 min
5) La composition est conservée à température ambiante

### Exemple 1b : Préparation des compositions mousseuses de polymère

La préparation des compositions mousseuses ne diffère que par une étape supplémentaire (étape 6) dans laquelle un gaz est ajouté.
1) Le polymère hydrosoluble est pesé en flacon de verre et dilué en eau déminéralisée
2) L'ensemble est mis sous agitation et chauffé afin de dissoudre complètement le polymère en solution
3) Si présence d'un tensioactif, il est ajouté à la solution par pipetage (si liquide) ou par pesé (si solide)
4) La solution est autoclavée en cycle liquide, avec un plateau à 120°C durant 16 min
5) La composition est conservée à température ambiante
6) La composition est ajouté dans un dispositif contenant du CO₂ afin de créer par mélange la composition mousseuse lors du dépôt.

### Exemple 2 : Test de familles de polymères hydrosolubles

Différentes familles de polymères hydrosolubles ont été testées pour les propriétés suivantes :
- Dissolution du polymère dans l'eau et obtention d'une solution homogène
- Capacité à former un film par séchage
- Décollement du film d'une surface (plastique polystyrène cristal, verre, acier inoxydable 316L)
- Hydrosolubilité du film au contact d'un diluant aqueux
- Arrachage puis détection d'au moins un microorganisme

Les polymères ont été préparés selon le protocole suivant :
1) Le polymère est pesé en flacon de verre et dilué en eau déminéralisée
2) L'ensemble est mis sous agitation et chauffé afin de dissoudre complètement le polymère en solution
3) Un tensioactif est ajouté à la solution par pipetage à la concentration de 0,5 % v/v
4) La solution est conservée à température ambiante

Les polymères hydrosolubles synthétiques ayant démontré l'ensemble des propriétés décrites ci-dessus sont préférentiellement :
- Poly(ethylene oxide) (PEO) d'un poids moléculaire moyen compris entre 100 000 et 8 000 000 g/mol (Sigma Aldrich ; ref : 181986, 372781, 372838) à des concentrations comprises entre 2 et 30 % (m/v)
- Poly(ethylene glycol) (PEG) d'un poids moléculaire moyen compris entre 600 et 20 000 g/mol à des concentrations comprises entre 2 et 30% (m/v)
- Poly(vinyl pyrrolidone) (PVP) d'un poids moléculaire moyen compris entre 10 000 et 1 300 000 g/mol (Sigma Aldrich; ref: PVP10, PVP360, 437190), à des concentrations comprises entre 2 et 30% (m/v)
- Poly(vinyl alcohol) (PVA ou PVOH) d'un poids moléculaire moyen compris entre 9 000 et 200 000 g/mol (Sigma Aldrich ; ref : 360627 ; Merck Millipore ; ref : 8.43866.1000, 8.43867.1000), à des concentrations comprises entre 2 et 50% (m/v)
- Poly(acrylic acid) (PAA) d'un poids moléculaire moyen compris entre 450 000 et 4 000 000 g/mol (Sigma Aldrich ; ref : 181285, 306231), à des concentrations comprises entre 1 et 2% (m/v)
- Poly(acrylamide) d'un poids moléculaire moyen compris entre 40 000 et 150 000 g/mol (Sigma Aldrich ; ref : 738743, 749222) à des concentrations comprises entre 2 et 20% (m/v)
- Poly(2-hydroxypropyl methacrylamide) d'un poids moléculaire moyen compris entre 30 000 et 50 000 g/mol (Sigma Aldrich ; ref : 804746) à des concentrations comprises entre 2 et 30% (m/v)
- Poly (2-ethyl-2-oxazoline) d'un poids moléculaire moyen de 5 000 g/mol (Sigma Aldrich ; ref : 773379) à des concentrations comprises entre 2 et 10% (m/v)

### Exemple 3 : Test de familles de tensioactifs

Différentes familles de tensioactifs ont été testées pour les propriétés suivantes :
- Dilution du tensioactif dans la solution de polymère et obtention d'une solution homogène
- Peu ou pas d'impact négatif sur la cohésion du film, l'hydrosolubilité du film, la formation d'un film par séchage
- Facilite le décollement du film d'une surface (plastique polystyrène cristal, verre, acier inoxydable 316L)
- Arrachage puis détection d'au moins 1 microorganisme

Les solutions de polymère comprenant un tensioactif ont été préparées selon le protocole suivant :
1) Le polymère est pesé en flacon de verre et dilué en eau déminéralisée
2) L'ensemble est mis sous agitation et chauffé afin de dissoudre complètement le polymère en solution
3) Le tensioactif est pesé (si sous forme solide) ou mesuré (si sous forme liquide) puis est ajouté à la solution à la concentration de 0,5% m/v ou 0,5 % v/v
4) La solution est conservée à température ambiante

Les familles de tensioactifs ayant démontré l'ensemble des propriétés décrites ci-dessus sont préférentiellement :
- Tensioactifs anioniques, tels que le sodium dodecyl sulfate (Sigma Aldrich, ref. L3771), l'acide cholique (Sigma Aldrich, ref. C1129) et le sodium de dicyclohexyl sulfosuccinate (Sigma Aldrich, ref. 86141)
- Tensioactifs cationiques, tel que le Benzalkonium chloride (Sigma Aldrich, ref. 12060)
- Tensioactifs non ioniques, tels que le diethylene glycol (Sigma Aldrich, ref. 93171 ), le polysorbate 80 (Acros Organics ; ref: 278630000) et la saponine (Sigma Aldrich, ref. 84510)
- Tensioactifs zwitterioniques, , tels que le 3 - (N,N-Dimethyltetradecylammonio) propanesulfonate (Sigma Aldrich, ref. 40772) et le CHAPS ou 3- [(3-Cholamidopropyl)dimethylammonio] - 1 -propanesulfonate hydrate (Sigma Aldrich, ref. C9426)

### Exemple 4 : Vitesse de séchage du polymère (base 25cm²)

Afin d'évaluer la vitesse à laquelle la composition de polymère hydrosoluble est capable de passer de l'état liquide et/ou visqueux à l'état solide sous forme de film, des essais ont été effectués dans différentes conditions.

La composition de polymère PVOH 30 % m/v + polysorbate 80 0,5 % v/v a été fabriquée selon l'exemple 1 puis stockée à température ambiante.

1 ml de solution est déposée sur une surface d'acier inoxydable 316L carrée de 5 cm X 5 cm soit 25 cm². Les dépôts ont été placés en étuve ventilée (30% de ventilation) à différentes températures ou sous hotte à flux laminaire à température ambiante.

Le temps nécessaire à ce que l'ensemble de la surface de polymère de 25cm² soit sous forme de film a été chronométré.

Les résultats sont montés dans le tableau suivant :

| Condition de séchage | Hotte à flux laminaire (TA) | Etuve 22,5°C | Etuve 32,5°C | Etuve 37°C |
|---|---|---|---|---|
| Vitesse de séchage moyen (en heures) | 1,23 | 2,50 | 1,72 | 2,06 |
| Ecart type | 0,06 | 0,45 | 0,36 | 0,14 |

### Conclusion :

On démontre que la vitesse de séchage du polymère hydrosoluble est plus dépendante de la ventilation que de la température (ventilation supérieure sous hotte à flux qu'en étuve).

La vitesse de séchage est comprise entre 1h30 et 3h, démontrant la rapidité du polymère à passer d'un état visqueux à un état solide.

### Exemple 5: Analyse rhéologique des compositions au cours du séchage

Afin de caractériser physiquement le comportement de la solution de polymère au cours du séchage des analyses rhéologiques ont été effectuées.

Une solution de polymère PVOH 10 % m/v + polysorbate 80 0,5 % v/v et une solution de polymère PVOH 10 % m/v + polysorbate 80 5 % v/v ont été fabriquées selon l'exemple 1 puis stockées à température ambiante.

L'étude rhéologique a été effectuée sur un rhéomètre DHR-2 (TA Instruments) possédant une plateforme inférieure (Peltier) permettant le maintien en température et une plateforme supérieure recouverte d'un « doughnut » en plastique. Ce couvercle en forme de doughnut permet un séchage lent et uniforme du gel selon un axe radial, assurant une homogénéité d'épaisseur du gel au cours de séchage.

Environ 1 ml de solution de polymère est déposé sur la plateforme inférieure du rhéomètre, puis la plateforme supérieure est lentement descendue au contact de la solution, pour obtenir un gap initial de 500 µm. L'expérience est réalisée à une température constante de 25°C.

La hauteur de la plateforme supérieure est contrôlée par le rhéomètre de façon à imposer à l'échantillon une force nulle constante, qui ne varie pas au cours du séchage du gel. La hauteur de l'échantillon (gap) peut être ainsi suivie au cours du temps.

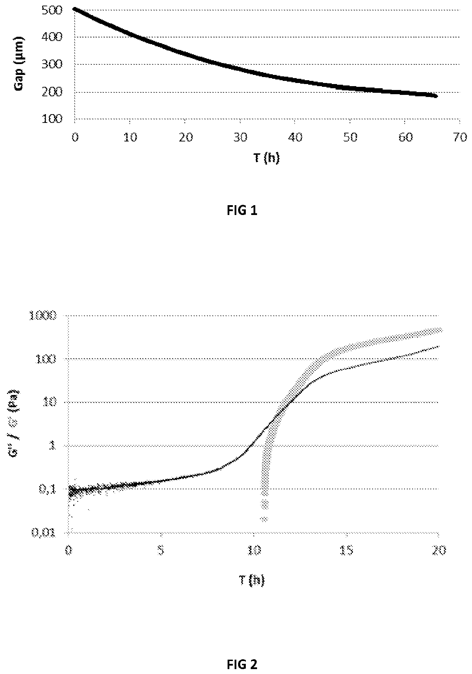

La figure 1 correspond à un graphique représentant la diminution de l'épaisseur de l'échantillon (solution de polymère PVOH 10 % m/v + polysorbate 80 5 % v/v) au cours du séchage.

La plateforme supérieure applique également des oscillations de cisaillement de faibles amplitudes (amplitude de contrainte : y = 0,1 % et fréquence = 1 Hz) permettant de mesurer les propriétés viscoélastiques de l'échantillon au cours du séchage.

Les propriétés viscoélastiques de l'échantillon se traduisent par le module visqueux ou module de perte (G'') et le module élastique ou module de conservation (G'). Lorsque le module élastique devient supérieur au module visqueux il est possible de déterminer la concentration en PVOH critique correspondant au point de gélification.

La figure 2 correspond à un graphique représentant l'évolution des modules visqueux (G") et élastique (G') au cours du séchage de la solution de polymère PVOH 10 % m/v + polysorbate 80 5 % v/v.

Au point de gélification, la concentration en PVOH critique obtenue est comprise entre 10 et 20 % m/v.

Le module élastique obtenu à la fin du séchage, et qui caractérise l'aspect solide du film de polymère, est supérieur à 100 000 Pa.

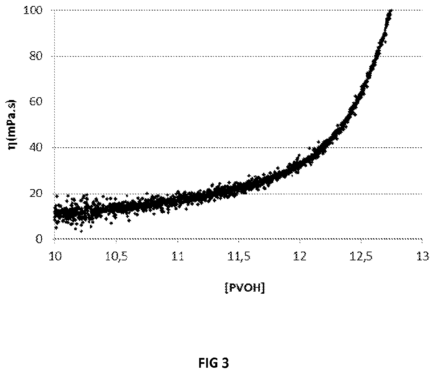

Finalement, la viscosité de la composition liquide et/ou visqueuse comprenant le polymère a été calculée en fonction de la concentration en polymère PVOH, représentée à la figure 3. Cette viscosité caractérise la solution de polymère sous sa forme liquide / visqueuse.

### Exemple 6 : Force d'arrachage du film

Afin de caractériser physiquement l'adhésion du film aux surfaces, des essais de force d'arrachage du film sur différentes surfaces ont été effectués.

Les solutions de polymères ont été fabriquées selon l'exemple 1 puis stockées à température ambiante. 1,5 g de solution sont déposés sur une surface rectangulaire d'une taille de 6,5 cm par 4 cm (26 cm²) puis séché 24 heures à température ambiante.

Après séchage et formation du film, une amorce d'environ 1 cm de film est décollée de la surface sur la largeur de 4 cm. Cette amorce est introduite dans la pince d'un banc de traction de MTS^{®} System, avec un angle d'environ 90°, à une distance de 6 mm. La vitesse d'arrachage est de 50 mm / min, sur une distance de 50 mm. La force moyenne nécessaire à l'arrachage du film est calculée par le banc de traction et présentée dans le tableau ci-dessous.

| **Composition** | **Surface** | **Force d'arrachage (en N)** | |
|---|---|---|---|
| | | **MOY** | **ET ( écart type)** |
| PVOH 30 % Polysorbate 80 0,5% | Plastique | 0,91 | 0,14 |
| PVOH 30 % Polysorbate 80 0,5% | Verre | 1,99 | 0,82 |
| PVOH 30 % Polysorbate 80 0,5% | Inox | 2,32 | 0,52 |
| PVOH 10 % Polysorbate 80 5% | Plastique | 0,39 | 0,03 |
| PVOH 50 % | Inox | 12,72 | 2,34 |

### Exemple 7 : Force d'élongation du film

Afin de caractériser la résistance du film à la déchirure, des essais de force d'élongation ont été effectués.

Les solutions de polymères ont été fabriquées selon l'exemple 1 puis stockées à température ambiante. 1g ou 3g de solution ont été déposés sur un gabarit rond d'une surface de 23 cm² puis séché 24 heures à température ambiante. Après décollement du film, un carré de 3 cm x 3 cm est découpé au centre du cercle pour assurer une homogénéité d'épaisseur de l'échantillon testé. Le film découpé est placé entre les deux pinces du banc de traction, à une distance de 10 mm. La vitesse d'élongation est de 10 mm / min jusqu'à rupture du film. La valeur retenue est la valeur maximale de force nécessaire à la rupture du film, visible sur la courbe d'élongation par un point d'inflexion net (chute brutale).

Les résultats sont présentés dans le tableau suivant :

| **Composition** | **Poids coulé** | **Epaisseur** | **Force d'élongation (en N)** | |
|---|---|---|---|---|
| | | | **MOY** | **ET** |
| PVOH 30 % polysorbate 0,5% | 1g | 44 µm | 38,76 | 3,65 |
| PVOH 30 % polysorbate 0,5% | 3g | 228 µm | 83,45 | 3,17 |
| PVOH 10 % polysorbate 0,5% | 1g | 56 µm | 12,75 | 3,98 |
| PVOH 10 % polysorbate 5% | 1g | 88 µm | 12,09 | 1,97 |
| PVOH 50 % polysorbate 0,5% | 1g | 111 µm | 70,11 | 5,30 |
| PVOH 50 % polysorbate 0,5% | 3g | 310 µm | 69,11 | 5,71 |
| PVOH 50 % polysorbate 5% | 1g | 113 µm | 53,14 | 7,40 |
| PVOH 50 % polysorbate 5% | 3g | 358 µm | 59,94 | 3,79 |

### Exemple 8 : Vitesse de dissolution du film de polymère (base 25cm²)

Afin d'évaluer la propriété d'hydrosolubilité du film de polymère PVOH, la vitesse de dissolution du film a été mesurée pour différent volume d'eau déminéralisée.

La solution de polymère PVOH 30 % m/v + polysorbate 80 0,5 % v/v a été fabriquée selon l'exemple 1 puis stockée à température ambiante.

1 ml de solution est déposée sur une surface plastique polystyrène cristal carrée de 5 cm X 5 cm soit 25 cm². L'ensemble est séché dans une étuve ventilée à 30%, à 37°C durant 1 heure, permettant à la solution de polymère de se solidifier sous forme de film.

Après séchage, le film de polymère est décollé de la surface plastique à l'aide d'une pince stérile. Le film est alors déposé dans un flacon contenant 100, 50, 10 ou 5 ml d'eau déminéralisée. Le flacon est agité à la main ou au vortex afin de dissoudre le film. Le temps nécessaire à la dissolution complète du film est chronométré.

Les résultats sont montrés dans le tableau suivant :

| | | | | |
|---|---|---|---|---|
| Volume de dissolution (ml) | 100 | 50 | 10 | 5 |
| Vitesse de dissolution moyenne (min) | 1,76 | 1,61 | 2,04 | 5,36 |
| Ecart type | 0,36 | 0,33 | 0,29 | 0,70 |

### Conclusion :

On démontre que la vitesse de dissolution du film de PVOH (1 ml) est identique pour un volume de dissolution de 100 ml à 10 ml ; soit 10 à 100 fois plus de volume d'eau déminéralisée que de volume de film déposé. En revanche le temps de dissolution augmente pour un volume de dissolution plus faible de 5 ml (soit 5 fois le volume de film déposé).

Finalement quel que soit le volume de dissolution testé, la dissolution du film de PVOH est très rapide, inférieure à 10 min même pour un volume très faible. Cela démontre l'excellente hydrosolubilité du film de PVOH, qui ne laisse aucune particule ou résidu après dissolution.

### Exemple 9 : Interêt du prélèvement de microorganisme à l'aide d'un polymère sous forme liquide et/ou visqueuse dans le cadre du procédé de la présente invention

Le prélèvement de microorganismes à l'aide d'un polymère sous forme liquide et/ou visqueuse dans le cadre du procédé de la présente invention a été comparé à un prélèvement à l'aide d'un polymère préalablement séché avant la mise en contact avec le microorganisme à prélever.

La solution de polymère PVOH 30 % m/v + polysorbate 80 0,5 % v/v a été fabriquée selon l'exemple 1 puis stockée à température ambiante.

### Dépôt sec de microorganismes :

Une quantité connue de *Staphylococcus aureus* est déposée sur une lame de verre (Rq ≈ 0,5nm). Pour cela une BioBall multishot 550 CFU (bioMérieux ; Ref: 56019) est diluée dans 500 µl de fluide de réhydratation (bioMérieux ; Ref: 56021) puis vortexée 30 secondes. 50µl de la solution de *Staphylococcus aureus* (environ 50 UFC [Unité Formant Colonie]) est déposé sur la lame de verre. Le dépôt est séché dans une étuve ventilée à 30%, à 37°C durant 30 minutes.

En parallèle, 50µl de la solution de *Staphylococcus aureus* est déposé sur des boites de Pétri Trypto-caséine soja (TSA) (bioMérieux ; Ref: 43011 et 43711). Ces boites témoins sont incubées à 30-35°C durant 48 heures.

### Dépôt du polymère :

1) Forme liquide : la solution de polymère PVOH + polysorbate 80 est déposée par pipetage sur le dépôt sec de microorganismes *Staphylococcus aureus,* puis étalée à l'oese stérile si nécessaire afin de recouvrir entièrement le dépôt sec. L'ensemble est séché dans une étuve ventilée à 30%, à 37°C durant 1 heure, permettant à la solution de polymère de se solidifier sous forme de film.
2) Forme solide : la solution de polymère PVOH + polysorbate 80 est déposée par pipetage sur une lame de verre puis étalée à l'oese stérile. La solution est séchée dans une étuve ventilée à 30%, à 37°C durant 1 heure, permettant à la solution de polymère de se solidifier sous forme de film. Le film obtenu est ensuite appliqué pendant 10 secondes et avec une force de 500 g sur le dépôt sec de Staphylococcus aureus.

### Décollement du polymère et détection des microorganismes :

Après séchage ou application directe, le film de polymère est décollé de la lame de verre à l'aide d'une pince stérile. Le film est alors déposé sur une boite de Pétri TSA (bioMérieux ; Ref: 43011 et 43811). Après quelques minutes le film s'est totalement solubilisé grâce à l'eau contenue dans la boite de Pétri. Les boites sont incubées à 30-35°C durant 48 heures.

### Comptage des microorganismes et analyse :

Après 48 heures d'incubation, les colonies présentes sur les boites témoins et les boites contenant le film de polymère hydrosolubilisé sont dénombrées. Le taux de récupération du polymère est calculé comme suit :

Taux de récupération : (100 X dénombrement TSA polymère) / (dénombrement TSA témoin) Les résultats sont présentés dans le tableau suivant :

| | Taux de récupération moyen (%) | Ecart type |
|---|---|---|
| Forme liquide | 56,2 | 0,14 |
| Forme solide | 18,7 | 0,08 |

### Conclusion :

Ces résultats démontrent que l'application de la solution de polymère hydrosoluble sous forme liquide et/ou visqueuse puis séchage pour obtenir un film permet de récupérer trois fois plus de microorganismes que l'application directe du film déjà sous forme solide.

### Exemple 10 : Intérêt du prélèvement de microorganisme à l'aide d'un polymère sous forme mousseuse dans le cadre du procédé de la présente invention

Le prélèvement de microorganismes à l'aide d'un polymère sous forme mousseuse dans le cadre du procédé de la présente invention a été comparé à une solution de polymère déposé sous forme liquide/visqueuse, sur une surface en inox (Rq ≈ 0,4µm).

### Dépôt sec de microorganismes :

Une quantité connue de *Staphylococcus aureus* est déposée sur une surface en inox. Pour cela une BioBall multishot 550 CFU (bioMérieux ; Ref: 56019) est diluée dans 500 µl de fluide de réhydratation (bioMérieux ; Ref: 56021) puis vortexée 30 secondes. 50µl de la solution de *Staphylococcus aureus* (environ 50 UFC [Unité Formant Colonie]) est déposé sur la surface en inox. Le dépôt est séché dans une étuve ventilée à 30%, à 37°C durant 30 minutes.

### Dépôt du polymère :

1) Témoin Forme liquide : la solution de polymère PVOH 10 % m/v + polysorbate 80 0,5 % v/v a été fabriquée selon l'exemple 1a. Cette solution est déposée par pipetage sur le dépôt sec de microorganismes *Staphylococcus aureus,* puis étalée à l'oese stérile si nécessaire afin de recouvrir entièrement le dépôt sec. L'ensemble est séché dans une étuve ventilée à 30%, à 37°C durant 1 heure, permettant à la solution de polymère de se solidifier sous forme de film.
2) Forme mousse : la mousse de polymère PVOH 10 % m/v + polysorbate 80 0,5 % v/v a été fabriquée selon l'exemple 1b. Cette mousse est déposée sur le dépôt sec de microorganismes *Staphylococcus aureus.* L'ensemble est séché dans une étuve ventilée à 30%, à 37°C durant 1 heure, la solution mousseuse passe à l'état liquide avant de se solidifier sous forme de film.

### Décollement du polymère et détection des microorganismes :

Après séchage, les films de polymère sont décollés de la surface inox à l'aide d'une pince stérile. Le film est alors déposé sur une boite de Pétri TSA (bioMérieux ; Ref: 43011 et 43811). Après quelques minutes le film s'est totalement solubilisé grâce à l'eau contenue dans la boite de Pétri. Les boites sont incubées à 30-35°C durant 48 heures.

### Comptage des microorganismes et analyse :

Après 48 heures d'incubation, les colonies présentes sur les surfaces et les boites contenant le film de polymère hydrosolubilisé sont dénombrées. Le taux de récupération du polymère est calculé comme suit :
Taux de récupération : (100 X dénombrement TSA polymère) / (dénombrement TSA témoin)

Les résultats sont présentés dans le tableau suivant :

| | Taux de récupération moyen (%) | Ecart type |
|---|---|---|
| Dépôt solution liquide | 18 | 0,03 |
| Dépôt mousse | 49 | 0,12 |

### Conclusion :

Sur une surface rugueuse telle qu'une surface en inox, l'application d'une solution mousseuse de polymère permet de récupérer environ 2,5 fois plus de microorganismes par rapport à une application de polymère sous forme liquide.

### Exemple 11 : Influence de la concentration en PVOH sur le taux de récupération

Afin d'étudier l'influence de la concentration en polymère PVOH sur le taux de récupération des microorganismes, une gamme de solution a été testée : de 10 à 50% m/v en PVOH avec 0,5% de polysorbate 80.

Les solutions ont été fabriquées selon l'exemple 1 puis stockées à température ambiante.

### Dépôt sec de microorganismes :

Une quantité connue de *Staphylococcus aureus* est déposée sur une lame de verre. Pour cela une BioBall multishot 550 CFU (bioMérieux ; Ref: 56019) est diluée dans 500 µl de fluide de réhydratation (bioMérieux ; Ref: 56021) puis vortexée 30 secondes. 50µl de la solution de *Staphylococcus aureus* (environ 50 UFC) est déposé sur la lame de verre. Le dépôt est séché dans une étuve ventilée à 30%, à 37°C durant 30 minutes.

En parallèle, 50µl de la solution de *Staphylococcus aureus* est déposé sur des boites de Pétri TSA (bioMérieux ; Ref: 43011 et 43711). Ces boites témoins sont incubées à 30-35°C durant 48 heures.

### Dépôt du polymère :

La solution de polymère PVOH + Polysorbate 80 est déposée par pipetage sur le dépôt sec de microorganismes *Staphylococcus aureus,* puis étalée à l'oese stérile si nécessaire afin de recouvrir entièrement le dépôt sec. L'ensemble est séché dans une étuve ventilée à 30%, à 37°C durant 1 heure, permettant à la solution de polymère de se solidifier sous forme de film.

### Décollement du polymère et détection des microorganismes :

Après séchage, le film de polymère est décollé de la lame de verre à l'aide d'une pince stérile. Le film est alors déposé sur une boite de Pétri TSA (bioMérieux ; Ref: 43011 et 43811). Après quelques minutes le film s'est totalement solubilisé grâce à l'eau contenue dans la boite de Pétri. Les boites sont incubées à 30-35°C durant 48 heures.

### Comptage des microorganismes et analyse :

Après 48 heures d'incubation, les colonies présentes sur les boites témoins et les boites contenant le film de polymère hydrosolubilisé sont dénombrées. Le taux de récupération du polymère est calculé comme suit :
Taux de récupération : (100 X dénombrement TSA polymère) / (dénombrement TSA témoin)

Les résultats sont montés dans le tableau suivant :

| | | | | | | | | | |
|---|---|---|---|---|---|---|---|---|---|
| Concentration en PVOH (avec 0,5% Polysorbate 80) en % m/v | 10 | 15 | 20 | 25 | 30 | 35 | 40 | 45 | 50 |
| Taux de récupération moyen (%) | 64% | 80% | 62% | 67% | 68% | 72% | 52% | 51% | 47% |
| Ecart type | 0,06 | 0,17 | 0,14 | 0,05 | 0,06 | 0,07 | 0,12 | 0,12 | 0,08 |

### Conclusion :

On démontre que la concentration en PVOH de la solution de polymère n'influence pas significativement le taux de récupération du *Staphylococcus aureus,* sauf au-delà de 35% m/v où le taux de récupération à tendance à diminuer. En deçà de 40% m/v de PVOH en solution, le taux de récupération du *Staphylococcus aureus* est compris entre 60 et 80%.

Finalement, ces taux de récupération démontrent un très bon enveloppement des microorganismes dans le film, puis un très bon arrachage par le film et aucune toxicité de la solution de PVOH + Polysorbate.

### Exemple 12 : Influence de la concentration en polysorbate 80 sur le taux de récupération

Afin d'étudier l'influence de la concentration en polysorbate 80 sur le taux de récupération des microorganismes, une gamme de solution ont été fabriquée selon l'exemple 1 : de 0 à 5% v/v en polysorbate 80 avec 30% m/v de PVOH.

Les solutions ont été testées selon le même protocole que l'exemple 11.

Les résultats sont montés dans le tableau suivant :

| | | | | | |
|---|---|---|---|---|---|
| Concentration en Polysorbate 80 (avec PVOH 30% m/v) en % v/v | 0 | 0,5 | 1 | 2 | 5 |
| Taux de récupération moyen (%) | 12% | 73% | 73% | 64% | 45% |
| Ecart type | 0,05 | 0,12 | 0,23 | 0,19 | 0,19 |

### Conclusion :

On démontre que la concentration en polysorbate 80 influence le taux de récupération des microorganismes. En l'absence de polysorbate 80, le taux de récupération du *Staphylococcus aureus* est fortement diminué. L'ajout de 0,5 ou 1% de polysorbate 80 permet d'obtenir un taux de récupération d'environ 70% qui diminue au-delà de cette concentration.

### Exemple 13: Détection sur boite de Pétri

La solution de polymère PVOH 30 % m/v + polysorbate 80 0,5 % v/v a été fabriquée selon l'exemple 1 puis stockée à température ambiante.

La solution de polymère est déposée sur la surface à échantillonner puis, après séchage, le film est retiré de la surface, à l'aide d'une pince stérile (cf protocole exemple 11).

Afin de détecter et de dénombrer le nombre de microorganismes présents dans le film, donc initialement sur la surface, le film est déposé sur milieu de culture solide. Très rapidement, l'eau contenue dans la gélose permet de solubiliser le film et celui-ci disparait totalement de la surface du milieu de culture. Différents milieux de culture solides peuvent être utilisés en fonction des exigences des microorganismes que l'on souhaite détecter.

Les milieux de culture sont ensuite incubés dans les conditions habituelles de culture. Après incubation, les colonies isolées peuvent être aisément dénombrées afin de rendre un résultat quantitatif du contrôle de surface.

Des essais sur boites de Pétri TSA (bioMérieux, ref : 43811) ont permis de démontrer que la taille des colonies à 24 / 48 / 72 heures, est équivalente entre un dépôt d'inoculum direct et un dépôt après arrachage du polymère hydrosoluble.

De plus, des essais ont été réalisés avec un milieu contenant un indicateur coloré (milieu Chapman ; bioMérieux, ref : 46671) et un milieu contenant un chromogène (milieu SAID ; bioMérieux, ref : 419042). Les résultats montrent que les colorations des colonies, du milieu, l'aspect des colonies sont équivalents entre un dépôt d'inoculum direct et un dépôt après arrachage du polymère hydrosoluble.

Ces résultats démontrent la compatibilité du polymère hydrosoluble avec les méthodes de croissance sur milieu de culture.

### Exemple 14 : Détection par méthodes rapide (BacT/ALERT^{®}, Tempo^{®}, Gene-Up^{®})

### BacT/ALERT^{®} :

La technologie BacT/ALERT^{®} est une méthode de détection des microorganismes cinétique par colorimétrie. Les micro-organismes présents dans le milieu produisent du CO₂ lors de leur phase de croissance. Le CO₂ produit induit une diminution du pH du milieu de culture. Ce changement de pH induit le virage colorimétrique d'un sensor composé de silicone imprégné de détecteurs d'émulsion liquide (LES), déposé au fond de chaque flacon. Une LED envoie un faisceau lumineux sur le sensor. Une photodiode collecte l'intensité de lumière réfléchie par le sensor sous la forme d'unité de réflectance. Les unités de réflectance sont analysées dans le temps.

La solution de polymère PVOH 30 % m/v + polysorbate 80 0,5 % v/v a été fabriquée selon l'exemple 1 puis stockée à température ambiante.

La solution de polymère est déposée sur une surface préalablement chargée avec une quantité connue du microorganisme *Staphylococcus aureus* (environ 50 UFC), tel que décrit dans l'exemple 9. L'ensemble est séché dans une étuve ventilée à 30%, à 37°C durant 1 heure, permettant à la solution de polymère de se solidifier sous forme de film. Après séchage, le film de polymère est décollé de surface l'aide d'une pince stérile. Le film est alors dilué dans 2 ml d'eau physiologique (bioMérieux, ref : 33892). Après solubilisation complète la solution est prélevée à la seringue puis injectée dans un flacon BacT/ALERT^{®} SA (bioMérieux, ref : 259789). En parallèle, des flacons témoins sont ensemencés directement avec la même quantité connue de la souche *Staphylococcus aureus* (environ 50 UFC). Les flacons sont chargés sur l'automate BacT/ALERT^{®} VIRTUO^{®} (bioMérieux ; ref: 411660) pour une lecture cinétique automatique.

Les résultats montrent des temps de détection et des courbes de croissance similaires entre les flacons témoins ensemencés directement avec la souche et les flacons ensemencés avec le film de polymère solubilisé. Ces résultats démontrent la compatibilité du polymère hydrosoluble avec une méthode de détection des microorganismes par colorimétrie.

### TEMPO^{®} :

La technologie Tempo est une méthode de dénombrement des microorganismes basée sur la méthode du nombre le plus probable (NPP) en analysant des séries de dilutions de l'échantillon initial. La technologie Tempo miniaturise ce test sur une carte conçue spécifiquement. Le système est composé de :
- Un poste de préparation (TEMPO^{®} FILLER) pour ensemencer les cartes TEMPO^{®} avec les échantillons et les milieux de culture spécifiques. Les milieux de culture TEMPO^{®} permettent le développement rapide des bactéries ou levures / moisissures et contiennent un indicateur fluorescent. La carte TEMPO^{®} , innovante, intègre une méthode NPP de 16x3 tubes miniaturisés.
- Un poste de lecture automatisé (TEMPO^{®} READER) pour déterminer dans chacune des carte la concentration bactérienne et ainsi déterminer le nombre de microorganismes présents dans l'échantillon initial.

La solution de polymère PVOH 30 % m/v + polysorbate 80 0,5 % v/v a été fabriquée selon l'exemple 1 puis stockée à température ambiante.

La solution de polymère est déposée sur une surface préalablement chargée avec une quantité connue du microorganisme *Staphylococcus aureus* (environ 50 UFC), tel que décrit dans l'exemple 9. L'ensemble est séché dans une étuve ventilée à 30%, à 37°C durant 1 heure, permettant à la solution de polymère de se solidifier sous forme de film. Après séchage, le film de polymère est décollé de la surface à l'aide d'une pince stérile. Le film est alors déposé dans un flacon TEMPO CTB (bioMérieux, ref : 416683). En parallèle, des flacons témoins sont ensemencés directement avec la même quantité connue de la souche *Staphylococcus aureus* (environ 50 UFC). Le milieu contenu dans le flacon est réhydraté en ajoutant 4 ml d'eau distillée par flacon, permettant également au film de polymère de se solubiliser.

Après réhydratation et solubilisation complète, les flacons TEMPO^{®} CTB puis les cartes TEMPO^{®} CTB correspondantes (bioMérieux, ref: 416683) sont scannés sur le poste TEMPO^{®} FILLER. Les flacons et cartes sont placés sur le rack de remplissage, placé dans la station de préparation qui va remplir complétement la carte puis couper les pailles et ainsi sceller la carte.

Les cartes sont incubées à 30°C ± 1°C durant 24h-28h (détection bactérienne).

Après incubation, les cartes sont placées dans la station de lecture TEMPO^{®} READER et lues automatiquement. Le résultat s'affiche directement en UFC/ml, en prenant en compte le facteur de dilution.

Les résultats montrent des dénombrement bactériens équivalents entre les flacons témoins ensemencés directement avec la souche et les flacons ensemencés avec le film de polymère solubilisé. Ces résultats démontrent la compatibilité du polymère hydrosoluble avec une méthode de détection des microorganismes par fluorescence en utilisant la méthode NPP .

| | Flacons témoins | Polymère sur verre | Polymère sur plastique |
|---|---|---|---|
| Dénombrement moyen au TEMPO^{®} (en UFC/ml) | 56 | 65 | 60 |
| Ecart type | 0 | 23,09 | 10,58 |

### Gene UP^{®} :

La technologie Gene-Up^{®} est une méthode de détection des microorganismes par PCR en temps réel. Suite à un enrichissement en milieu de culture, l'ADN des microorganismes est libéré par lyse mécanique, puis détecté par PCR en temps réel via des sondes FRET et des pics de fusion de sondes pour garantir la spécificité du test.

La solution de polymère PVOH 30 % m/v + polysorbate 80 0,5 % v/v a été fabriquée selon l'exemple 1 puis stockée à température ambiante.

Différentes surfaces sont chargées en microorganisme Listeria monocytogenes puis séchées dans une étuve ventilée à 30%, à 37°C durant 30 minutes pour obtenir des dépôts secs. La solution de polymère est alors déposée sur les microorganismes puis séchée dans une étuve ventilée à 30%, à 37°C durant 1 heure, permettant à la solution de polymère de se solidifier sous forme de film. Après séchage, le film de polymère est décollé de la surface à l'aide d'une pince stérile puis déposé dans un tube de milieu d'enrichissement LPT (bioMérieux, ref : 410845). En parallèle, des dépôts secs de microorganismes sont échantillonnés avec un écouvillon (bioMérieux, ref: 70.6016) ensuite déposés dans un tube de milieu d'enrichissement LPT. Les tubes sont incubés à 37°C durant 18-24 heures.

Après enrichissement, 20µl d'échantillon sont déposés dans un tube de lyse (bioMérieux, Gene-Up^{®} lysis kit, ref : 414057). Les tubes de lyse sont placés sur un portoir et agités au vortex Troemner à 2200 tr/min pendant 5 minutes.

Un flacon de réactif lyophilisé (bioMérieux, Kit Listeria spp : ref : 414059, Kit listeria monocytogenes : ref : 414058) est reconstitué avec 45 µl de tampon de reconstitution. Les tubes de PCR vides sont placés sur un portoir et 5µl de réactif PCR sont déposés dans chaque tube. Sont ensuite ajoutés à chaque tube 5µl d'échantillon lysé. Un témoin négatif constitué de 5µl de tampon de contrôle est effectué pour chaque kit. Les tubes sont fermés à l'aide de bouchons, scellés, puis le portoir est centrifugé. La plaque est placée dans l'instrument Gene UP.

Les échantillons et les kits de détection associés sont renseignés dans le logiciel Gene UP avant démarrage. Les résultats sont automatiquement interprétés quand le cycle PCR est terminé. Le logiciel interprète les données des courbes d'amplification et de fusion pour chaque échantillon et donne un résultat positif, négatif ou inhibé.

Les résultats démontrent des détections équivalentes entre les échantillons témoins et les échantillons prélevés avec le polymère pour toutes les surfaces testées. De plus, tous les contrôles internes à la PCR sont détectés.

Ces résultats démontrent la compatibilité du polymère avec une méthode de détection via la technique de PCR. Notamment le polymère n'inhibe pas la réaction de PCR.

| **Kit de détection** | **Réplicat** | **Témoin plastique** | **Polymère plastique** | **Témoin verre** | **Polymère verre** | **Contrôle négatif** |
|---|---|---|---|---|---|---|
| *Listeria spp* | 1 | Positif | Positif | Positif | Positif | Négatif |
| | 2 | Positif | Positif | Positif | Positif | |
| *Listeria monocytogenes* | 1 | Positif | Positif | Positif | Positif | Négatif |
| | 2 | Positif | Positif | Positif | Positif | |

### Exemple 15 : Détection par méthode biochimique (VIDAS^{®})

La technologie VIDAS^{®} est une méthode de détection d'éléments (hormone, virus, microorganismes ..) par immuno-essai. Le principe VIDAS^{®} consiste en l'interaction de deux éléments : le cône (phase solide) dont la surface interne est recouverte d'antigènes ou d'anticorps, et les Barrettes, composées de plusieurs puits et contenant la quantité exacte de réactifs nécessaires pour le test.

Les réactions se produisent dans le cône en deux étapes clés :
- réaction immunologique, capturant l'élément recherché ;
- réaction enzymatique, permettant de révéler la présence de l'élément recherché par la technique ELFA (Enzyme Linked Fluorescent Assay).

L'opération complète est automatisée : de l'incubation, aux lavages et à la lecture finale. Le temps d'incubation et le nombre de cycle de lavage sont optimisés pour chaque paramètre afin d'assurer une performance optimale.

La solution de polymère PVOH 30 % m/v + polysorbate 80 0,5 % v/v a été fabriquée selon l'exemple 1 puis stockée à température ambiante.

Différentes surfaces sont chargées en microorganisme Listeria monocytogenes puis séchées dans une étuve ventilée à 30%, à 37°C durant 30 minutes pour obtenir des dépôts secs. La solution de polymère est alors déposée sur les microorganismes puis séchée dans une étuve ventilée à 30%, à 37°C durant 1 heure, permettant à la solution de polymère de se solidifier sous forme de film. Après séchage, le film de polymère est décollé de la surface à l'aide d'une pince stérile puis déposé dans un tube de milieu d'enrichissement LPT (bioMérieux, ref : 410845). En parallèle, des dépôts secs de microorganismes sont échantillonnés avec un écouvillon (bioMérieux, ref: 70.6016) ensuite déposés dans un tube de milieu d'enrichissement LPT. Les tubes sont incubés à 30°C durant 22-30 heures.

Après enrichissement, 500µl d'échantillon sont déposés dans le puits échantillon d'une barrette VIDAS^{®} UP Listeria (bioMérieux, ref: 30126). La barrette est chauffée 5 minutes à 95-100°C sur l'appareil VIDAS^{®} Heat and Go puis laissée refroidie 10 minutes.

Comme contrôle positif, deux standards S1 sont réalisés en déposant 500µl de standard S1 dans le puits échantillon d'une barrette VIDAS^{®} UP Listeria (bioMérieux, ref : 30126). Ces barrettes ne sont pas chauffées.

Les cônes et barrettes VIDAS^{®} sont placés dans l'instrument, les échantillons et le paramètre de détection associé sont renseignés dans le logiciel avant démarrage du test. Les résultats sont obtenus en 62 minutes environ.

Les résultats montrent des positivités équivalentes entre les échantillons témoins et les échantillons prélevés avec le polymère pour toutes les surfaces testées. Ces résultats démontrent la compatibilité du polymère hydrosoluble avec une méthode de détection par immuno-essai.

### Exemple 16 : Identification par méthode biochimique (Vitek 2)

Le VITEK^{®} 2 compact est un système d'identification microbien basé sur des réactions biochimiques colorimétriques miniaturisées sur une carte conçue spécifiquement. Toutes les phases d'identification, de la lecture à l'enregistrement des résultats, sont automatisées. Le VITEK^{®} 2 Compact comprend une base de données d'identification étendue qui permet d'identifier de nombreux micro-organismes.

La solution de polymère PVOH 30 % m/v + polysorbate 80 0,5 % v/v a été fabriquée selon l'exemple 1 puis stockée à température ambiante.

La solution de polymère est déposée sur une surface préalablement chargée avec une colonie de du microorganisme *Kocuria kristinae.* L'ensemble est séché dans une étuve ventilée à 30%, à 37°C durant 1 heure, permettant à la solution de polymère de se solidifier sous forme de film. Après séchage, le film de polymère est décollé de la surface à l'aide d'une pince stérile. Le film est placé dans un tube à hémolyse puis solubilisé en ajoutant 3 ml de solution saline (biomérieux ; ref : 21218) pour VITEK^{®}. En parallèle, un tube témoin contenant 3 ml de solution saline est préparé pour obtenir 0,5 McFarland de la souche *Kocuria kristinae.* Les tubes sont chargés avec une carte VITEK^{®} GP (bioMérieux ; ref : 21342) dans le portoir Smart Carrier Station^{™}. Une fois les cassettes chargées, le système gère l'incubation et la lecture de chaque carte sans aucune autre intervention. Les résultats sont obtenus dans les 2 à 18 heures après chargement.

Les résultats montrent des identifications équivalentes (« Excellente identification ») entre le tube témoin ensemencé directement avec la souche et le tube ensemencé avec le film de polymère solubilisé. Ces résultats démontrent la compatibilité du polymère hydrosoluble avec une méthode d'identification biochimique colorimétrique.

## Revendications

1. Procédé de détection et/ou d'identification d'au moins un microorganisme cible présent sur une surface, comprenant les étapes suivantes :
a) déposer sur ladite surface, une composition comprenant un polymère synthétique hydrosoluble filmogène, ladite composition étant une composition liquide et/ou visqueuse ou une composition mousseuse ;
b) sécher la composition pour permettre la formation d'un film de polymère,
c) retirer de la surface ledit film de polymère comprenant ledit au moins un microorganisme cible,
d) dissoudre le film polymère avec un diluant aqueux pour former une solution comprenant ledit au moins un microorganisme, ledit diluant aqueux étant un milieu de culture semi-solide,
e) détecter et/ou identifier ledit au moins un microorganisme cible dans tout ou partie de la solution à l'aide d'au moins un moyen de détection.

2. Procédé selon la revendication 1 dans laquelle le moyen de détection est le milieu de culture dans lequel a été dissout le film polymère.

3. Procédé selon l'une quelconque des revendications précédentes dans laquelle la surface est une surface inerte.

4. Procédé selon l'une quelconque des revendications 1 à 3 **caractérisé en ce que** ledit procédé comprend une étape d'incubation du milieu de culture, avant ou pendant l'étape de détection, à une température et durant un laps de temps suffisant pour permettre la croissance dudit au moins un micro-organisme.

5. Procédé selon l'une quelconque des revendications précédentes **caractérisé en ce que** le polymère est choisi parmi l'oxyde de polyéthylène, le polyéthylène glycol, le polyvinylpyrrolidone, l'alcool polyvinylique, l'acide polyacrylique, le polyacrylamide, le poly(2-hydroxypropyl methacrylamide), le poly (2-ethyl-2-oxazoline).

6. Procédé selon la revendication 5 **caractérisé en ce que** le polymère est de l'alcool polyvinylique d'un poids moléculaire moyen compris entre 9 000 et 200 000 g/mol et à une concentration comprise entre 2 et 60% (m/v) préférentiellement entre 5 et 50% (m/v) encore plus préférentiellement entre 15 et 35% (m/v).

7. Procédé selon l'une quelconque des revendications précédentes **caractérisé en ce que** la composition comprend un tensioactif.

8. Procédé selon la revendication 7 **caractérisé en ce que** le tensioactif est choisi parmi le sodium dodecyl sulfate, l'acide cholique, le sodium de dicyclohexyl sulfosuccinate, le Benzalkonium chloride, le diethylene glycol, le polysorbate 80, la saponine, le 3 - (N,N-Dimethyltetradecylammonio) propanesulfonate, le 3-[(3-Cholamidopropyl)dimethylammonio]-1-propanesulfonate hydrate.

9. Procédé selon la revendication 8 **caractérisé en ce que** le tensioactif est du polysorbate 80 à une concentration égale ou inférieure à 5%, préférentiellement entre 0,5 à 1%.

## Patentansprüche

1. Verfahren zum Nachweis und/oder zur Identifizierung mindestens eines auf einer Oberfläche vorhandenen Zielmikroorganismus, das die folgenden Schritte umfasst:
a) das Abscheiden einer Zusammensetzung auf der Oberfläche, die ein filmbildendes wasserlösliches synthetisches Polymer umfasst, wobei es sich bei der Zusammensetzung um eine flüssige und/oder viskose Zusammensetzung oder eine Schaumzusammensetzung handelt;
b) das Trocknen der Zusammensetzung, wodurch die Bildung eines Polymerfilms ermöglicht wird,
c) das Entfernen des den mindestens einen Zielmikroorganismus umfassenden Polymerfilms von der Oberfläche,
d) das Lösen des Polymerfilms mit einem wässrigen Verdünnungsmittel, wodurch eine den mindestens einen Mikroorganismus umfassende Lösung gebildet wird, wobei es sich bei dem wässrigen Verdünnungsmittel um ein halbfestes Kulturmedium handelt,
e) das Nachweisen und/oder Identifizieren des mindestens einen Zielmikroorganismus in der gesamten Lösung oder einem Teil davon mit mindestens einem Nachweismittel.

2. Verfahren nach Anspruch 1, wobei es sich beim Nachweismittel um das Kulturmedium handelt, in dem der Polymerfilm gelöst worden ist.

3. Verfahren nach einem der vorhergehenden Ansprüche, wobei es sich bei der Oberfläche um eine inerte Oberfläche handelt.

4. Verfahren nach einem der Ansprüche 1 bis 3, **dadurch gekennzeichnet, dass** das Verfahren einen Schritt einer Inkubation des Kulturmediums vor oder während des Nachweisschrittes bei einer Temperatur und für einen Zeitraum umfasst, die ausreichend sind, um ein Wachstum des mindestens einen Mikroorganismus zu ermöglichen.

5. Verfahren nach einem der vorhergehenden Ansprüche, **dadurch gekennzeichnet, dass** das Polymer aus Polyethylenoxid, Polyethylenglycol, Polyvinylpyrrolidon, Polyvinylalkohol, Polyacrylsäure, Polyacrylamid, Poly(2-hydroxypropylmethacrylamid), Poly(2-ethyl-2-oxazolin) ausgewählt ist.

6. Verfahren nach Anspruch 5, **dadurch gekennzeichnet, dass** es sich beim Polymer um Polyvinylalkohol mit einer mittleren Molmasse zwischen 9000 und 200 000 g/mol und einer Konzentration zwischen 2 und 60 % (m/V), vorzugsweise zwischen 5 und 50 % (m/V), noch stärker bevorzugt zwischen 15 und 35 % (m/V) handelt.

7. Verfahren nach einem der vorhergehenden Ansprüche, **dadurch gekennzeichnet, dass** die Zusammensetzung ein Tensid umfasst.

8. Verfahren nach Anspruch 7, **dadurch gekennzeichnet, dass** das Tensid aus Natriumdodecylsulfat, Cholsäure, Natriumdicyclohexylsulfosuccinat, Benzalkoniumchlorid, Diethylenglycol, Polysorbat 80, Saponin, 3-(N,N-Dimethyltetradecylammonio)propansulfonat, 3-[(3-Cholamidopropyl)dimethylammonio]-1-propansulfonathydrat ausgewählt ist.

9. Verfahren nach Anspruch 8, **dadurch gekennzeichnet, dass** es sich beim Tensid um Polysorbat 80 mit einer Konzentration gleich oder niedriger als 5 %, vorzugsweise zwischen 0,5 und 1 %, handelt.

## Claims

1. Method for detecting and/or identifying at least one target microorganism present on a surface, comprising the following steps:
a) depositing on said surface a composition comprising a film-forming water-soluble synthetic polymer, said composition being a liquid and/or viscous composition or a foam composition;
b) drying the composition to allow the formation of a polymer film,
c) removing from the surface said polymer film comprising said at least one target microorganism,
d) dissolving the polymer film with an aqueous diluent to form a solution comprising said at least one microorganism, said aqueous diluent being a semisolid culture medium,
e) detecting and/or identifying said at least one target microorganism in all or part of the solution using at least one detection means.

2. Method according to Claim 1, wherein the detection means is the culture medium in which the polymer film has been dissolved.

3. Method according to either one of the preceding claims, wherein the surface is an inert surface.

4. Method according to any one of Claims 1 to 3, **characterized in that** said process comprises a step of incubating the culture medium, before or during the detection step, at a temperature and for a period of time sufficient to allow the growth of said at least one microorganism.

5. Method according to any one of the preceding claims, **characterized in that** the polymer is selected from polyethylene oxide, polyethylene glycol, polyvinylpyrrolidone, polyvinyl alcohol, polyacrylic acid, polyacrylamide, poly(2-hydroxypropyl methacrylamide), poly(2-ethyl-2-oxazoline).

6. Method according to Claim 5, **characterized in that** the polymer is polyvinyl alcohol with an average molecular weight comprised between 9 000 and 200 000 g/mol and at a concentration comprised between 2% and 60% (m/v), preferentially between 5% and 50% (m/v), even more preferentially between 15% and 35% (m/v).

7. Method according to any one of the preceding claims, **characterized in that** the composition comprises a surfactant.

8. Method according to Claim 7, **characterized in that** the surfactant is selected from sodium dodecyl sulfate, cholic acid, dicyclohexyl sulfosuccinate sodium, benzalkonium chloride, diethylene glycol, polysorbate 80, saponin, 3-(N,N-dimethyltetradecylammonio)propanesulfonate, 3-[(3-cholamidopropyl)dimethylammonio]-1-propanesulfonate hydrate.

9. Method according to Claim 8, **characterized in that** the surfactant is polysorbate 80 at a concentration equal to or less than 5%, preferentially between 0.5% and 1%.
